# FASCICULE DE BREVET EUROPEEN

(11) **EP 1 216 411 B1**
(45) Date de publication et mention de la délivrance du brevet: **30.12.2009**
(21) Numéro de dépôt: 00966234.7
(22) Date de dépôt: 29.09.2000
(51) Int. Cl.: G01N 21/17, C12Q 1/68

(54) **PROCEDE DE DETECTION D'UNE REACTION DE RECONNAISSANCE MOLECULAIRE**
VERFAHREN ZUM NACHWEIS EINER REAKTION ZUR MOLEKULAREN ERKENNUNG
METHOD FOR DETECTING A MOLECULAR RECOGNITION REACTION

(30) Priorité: 30.09.1999 FR 9912229
(43) Date de publication de la demande: 26.06.2002
(73) Titulaire: COMMISSARIAT A L'ENERGIE ATOMIQUE, 75015 Paris (FR); Biomerieux S.A., 69280 Marcy l'Etoile (FR)
(72) Inventeur: CHATON, Patrick, F-38570 Theys (FR); POUPINET, Ludovic, F-38170 Seyssinet (FR); GINOT, Frédéric, F-38340 Voreppe (FR); NOVELLI ROUSSEAU, Armelle, F-38180 Seyssins (FR)
(74) Mandataire: Audier, Philippe André
(86) Numéro de dépôt international: PCT/FR2000/002703
(87) Numéro de publication internationale: WO 2001/023867

(56) Documents cités:
- US-A- 5 874 213
- ADELHELM K ET AL: "Development of a sensitive detection system based on the photothermal effect for biomolecular interaction studies" BIOMEDICAL OPTOELECTRONICS IN CLINICAL CHEMISTRY AND BIOTECHNOLOGY;BARCELONA, SPAIN SEP 14-15 1995, vol. 2629, 14 septembre 1995 (1995-09-14), pages 325-333, XP000925207 Proc SPIE Int Soc Opt Eng;Proceedings of SPIE - The International Society for Optical Engineering 1996 Society of Photo-Optical Instrumentation Engineers, Bellingham, WA, USA
- ODAKE TAMAO ET AL: "High-speed separation using miniaturized slab gel and high spatial resolution detection by thermal lens microscope" PROCEEDINGS OF THE 1998 THERMAL THERAPY, LASER WELDING, AND TISSUE INTERACTION;STOCKHOLM, SWE SEP 9-SEP 11 1998, vol. 3565, 9 septembre 1998 (1998-09-09), pages 126-133, XP000925278 Proc SPIE Int Soc Opt Eng;Proceedings of SPIE - The International Society for Optical Engineering 1999 Society of Photo-Optical Instrumentation Engineers, Bellingham, WA, USA
- KITAMORI T: "Chemistry and analysis in integrated chemistry lab on chip" DIGEST OF PAPERS. MICROPROCESSES AND NANOTECHNOLOGY '99. 1999 INTERNATIONAL MICROPROCESSES AND NANOTECHNOLOGY CONFERENCE, DIGEST OF PAPERS. MICROPROCESSES AND NANOTECHNOLOGY '99. 1999 INTERNATIONAL MICROPROCESSES AND NANOTECHNOLOGY CONFERENCE, YOKOHA, pages 70-71, XP000925538 1999, Tokyo, Japan, Japan Society of Applied Physics, Japan ISBN: 4-930813-97-2

## Description

### Domaine technique de l'invention

La présente invention se rapporte à un procédé de détection sans marquage d'une réaction de reconnaissance moléculaire.

Cette invention concerne le domaine général de la détection et de l'analyse d'une reconnaissance moléculaire entre une première et une deuxième molécules, par exemple en biologie moléculaire.

Selon l'invention, la reconnaissance moléculaire peut être définie comme une interaction spécifique entre deux molécules plus ou moins complexes, conduisant à une liaison des deux molécules suffisamment stable pour que les molécules puissent être détectées liées. Il peut s'agir par exemple d'une hybridation d'acides nucléiques (ADN et/ou ARN), d'une réaction de reconnaissance de type antigène/anticorps, d'une interaction de type protéine/protéine, d'une interaction de type enzyme/substrat, etc... Les molécules concernées par la présente invention sont donc celles qui interviennent dans les interactions précitées.

Le procédé de la présente invention permet de détecter ce type de reconnaissance moléculaire, l'une des deux ou les deux molécules étant fixées, sur un support solide, en détectant une variation d'absorption par une méthode de photothermique.

Ce procédé trouve notamment une application dans la détection d'hybridation d'acides nucléiques sur support solide, en milieu aqueux ou à l'air, par exemple dans le cadre d'un criblage ("screening") ou d'une détection d'hybridation sur une biopuce.

### Etat de la technique

Par exemple la détection de l'hybridation d'acides nucléiques est généralement réalisée au moyen d'un marquage à l'aide d'une molécule fluorescente. Mais ce type de détection nécessite l'utilisation de plusieurs réactifs chimiques, et entraîne un coût et un temps de traitement élevés. De plus, les techniques utilisant la fluorescence ne sont pas toujours très précises. Enfin, il y a modification d'au moins une molécule par le marquage, ce qui peut affecter la réaction de reconnaissance moléculaire.

Aussi, d'autres méthodes ont été proposées par exemple des méthodes optiques consistant à détecter des variations d'épaisseur ou d'indice dans un échantillon par exemple par ellipsométrie, photogoniométrie, ou par des méthodes de résonance.

Cependant, par exemple dans le cas des oligonucléotides sur support solide, les couches formées sont ultra-fines, de quelques angströms à quelques nanomètres d'épaisseur, de sorte que la détection des variations d'épaisseur ou d'indice nécessite des appareillages de laboratoire extrêmement sensibles et chers.

La publication "Development of a sensitive detection system based on the photothermal effect for biomolecular interaction studies", ADELHELM K et AL, BIOMEDICAL OPTOELECTRONICS IN CLINICAL CHEMISTRY, VOL. 2629, 14 septembre 1995, pages 325-333, et la demande de brevet US-A-5874213 divulguent des procédés de détection photothermique de réaction entre molécules. Ces procédures nécessitent un marquage des molécules.

Il est aussi connu par la publication "High-speed séparation using miniaturized slab gel and high spatial resolution detection by thermal lens microscope", ODAKE TAMAO et AL, PROCEEDINGS OF THE 1998 THERMAL THERAPY, LASER WELDING, AND TISSUE INTERACTIONS, VOL. 3565, 9 septembre 1998, pages 126-133, de détecter par une méthode photothermique sans marquage des fragments d'ADN séparés sur un gel d'électrophorèse.

### Exposé de l'invention

La présente invention a précisément pour but de fournir une nouvelle technique de détection, sans marquage, d'une réaction de reconnaissance moléculaire entre une première molécule, dite de capture, fixée sur un support solide et une deuxième molécule, dite cible, présente dans une solution à tester.

Le procédé de l'invention est caractérisé en ce que la détection est réalisée par une méthode photothermique.

Selon l'invention, la réaction de reconnaissance ainsi que la première et la deuxième molécules peuvent être celles précitées.

Lorsque la première et la deuxième molécules sont des molécules d'acides nucléiques, le procédé de l'invention peut par exemple comprendre les étapes suivantes :
- fixation de la première molécule d'acide nucléique sur un support solide,
- mise en contact de la première molécule d'acide nucléique fixée sur le support solide avec une solution à tester soupçonnée de comprendre la deuxième molécule d'acide nucléique, cette dernière étant apte à s'hybrider à ladite première molécule, la mise en contact étant réalisée dans des conditions favorables à ladite hybridation,
- lavage du support solide pour isoler un échantillon de mesure constitué de ladite première molécule fixée sur le support et éventuellement de ladite deuxième molécule hybridée sur la première molécule, et
- mesure de l'absorption de l'échantillon par une méthode photothermique.

Le procédé de la présente invention peut comprendre en outre une étape de comparaison de la mesure de l'absorption de l'échantillon de mesure, précédemment défini avec celle d'un échantillon témoin dit de calibration, dont l'absorption est une mesure connue.

La première molécule, fixée sur le support, peut aussi être appelée molécule de capture, et la deuxième molécule, présente dans la solution à tester, peut aussi être appelée molécule cible. Lorsqu'il s'agit d'une molécule d'ADN fixée sur le support, elle peut aussi être appelée "sonde".

Il est important de noter que les couches minces d'acides nucléiques, hybridées ou non, sont habituellement considérées comme étant non absorbantes. Ceci est notamment décrit dans "Ellipsometric and interferometric characterization of DNA probes immobilized on a combinational assay", Gray et al., Langmuir 1997, 13, 2833-2842.

Malgré cela, les présents inventeurs se sont intéressés aux méthodes photothermiques appliquées à la détection et à l'analyse d'une réaction de reconnaissance moléculaire.

Ces méthodes ont toutes en commun l'excitation de l'échantillon dont l'absorption doit être mesurée par une source lumineuse, appelée faisceau pompe, en général un laser. Une partie de l'énergie lumineuse incidente est absorbée par l'échantillon. La proportion d'énergie absorbée est fixée par le spectre d'absorption de l'échantillon et le spectre d'émission de la source d'excitation. Une partie de l'énergie absorbée est transformée en chaleur. Le reste peut être rayonné ou donner naissance à de la fluorescence ou à une réaction chimique par exemple. La chaleur induit une variation de température dans le milieu absorbant et dans les milieux adjacents, cette variation de température peut également se traduire par une variation de densité et donc d'indice, ou par une variation de pression ou par l'apparition d'une onde acoustique. L'élévation de température due à l'absorption est en général inhomogène et donne donc lieu à un gradient d'indice du milieu analysé et des milieux adjacents, la densité des milieux étant rendue inhomogène par l'élévation de température.

Les méthodes photothermiques consistent à mesurer les effets induits par l'absorption.

Les inventeurs ont mis en évidence que parmi les méthodes photothermiques, la méthode de déflexion photothermique et la méthode de lentille thermique peuvent par exemple être utilisées selon la présente invention.

La méthode de déflexion photothermique est une méthode qui consiste à mesurer la déviation d'un faisceau lumineux, appelé faisceau sonde, passant dans la zone où se trouve le gradient d'indice. En d'autres termes, elle consiste à mesurer la déviation du faisceau sonde due à l'échauffement d'un échantillon absorbant par l'intermédiaire du faisceau pompe. Cette technique de déflexion photothermique a été appliquée à l'analyse de surface telle que la cartographie d'absorption, l'imagerie de paramètre thermique, mais jamais pour détecter une reconnaissance moléculaire telle que définie ci-dessus.

La méthode de lentille thermique consiste quant à elle à mesurer la variation de focalisation d'un faisceau lumineux passant dans la zone où se trouve le gradient d'indice.

Une présentation complète des méthodes photothermiques peut être trouvée par exemple dans l'ouvrage "Photothermal Spectroscopy Methods for Chemical Analysis, S.E. Bialkowski, vol. 134 in Chemical Analysis : a Series of Monographs on Analytical Chemistry and its Applications, Wiley".

Selon le procédé de l'invention, le support solide peut être défini comme étant un support sur lequel il est possible de fixer chimiquement directement ou indirectement la première molécule. Le support a de préférence une absorption faible vis-à-vis de celle de l'échantillon placé à sa surface afin de ne pas gêner la détection photothermique. Par ailleurs, il a de préférence une conductivité thermique faible car une conductivité trop importante du support induit une répartition large de la chaleur, donc une baisse du gradient de température et une baisse du signal détecté.

Selon l'invention, ce support peut être par exemple un support qui sert dans la fabrication des puces à ADN, par exemple un support de silice, de verre ou de plastique. Il est également possible de travailler avec des semi-conducteurs, en déposant sur leur surface des couches diélectriques pour améliorer le rapport signal à bruit.

Selon l'invention, la première molécule dite de capture est fixée sur le support. Cette fixation de la première molécule sur le support peut être réalisée par des réactions chimiques classiques, bien connues de l'homme du métier, et choisies en fonction du support, de la molécule à fixer, et des propriétés de résistance de la liaison que l'on désire pour l'application visée.

Cette fixation peut être directe ou indirecte.

Les documents 1 à 10 cités ci-dessous sont détaillés dans les références à la fin de cette description.

Par exemple, selon l'invention, lorsque le support est un support de silice, un traitement de fonctionnalisation du support de silice peut être réalisé, avant la fixation de la première molécule, de manière à modifier la surface du support pour y fixer des groupements chimiques réactifs qui permettront la fixation de la première molécule. Un tel traitement est décrit dans l'exemple 1 ci-dessous, mais on en trouvera de nombreux autres exemples dans la littérature spécialisée, comme Pease et al.(document 9), Guo et al. (document 4), Maskos et al. (document 6), etc. Comme il est dit précédemment, la silice n'est pas le seul support possible, et chaque support subira le traitement de surface qui lui convient. Pour illustrer ceci sur un film de polypropylène par exemple, on se référera à Matson et al. (document 7).

Les groupements chimiques réactifs sont généralement des groupements amine tels que ceux cités dans Guo et al. (document 4), ou carboxylique tels que ceux cités danzs Kohsaka et al. (document 5), ou époxy tels que ceux cités dans Maskos et al. (document 6), pour lesquels il existe des agents de couplage commerciaux efficace et facile d'utilisation (voir catalogue Pierce par exemple).

Le couplage covalent n'est pas la seule façon de fixer la première molécule sur son support. L'adsorption passive, largement utilisée pour fixer des anticorps sur des puits de réaction en polystyrène ou des microbilles tels que décrits dans Elaïssari et al. (document 3), est souvent très efficace voir aussi Balladur et al. (document 1). On peut aussi envisager des techniques de fixation plus récentes, comme la formation d'un film de Langmuir-Blodgett dans lequel est inclut des chaînes lipidiques sur lesquelles a été couplée chimiquement la première molécule voir Noy et al. (document 8) ; et Cornell et al. (document 2), ou encore la méthode métal-chélate voir Porath et al.,1975 (document 10).

Selon l'invention, lorsque la première molécule est fixée sur le support, les étapes suivantes peuvent être l'étape de mise en contact et l'étape de lavage précitées.

Selon l'invention, l'étape de mise en contact est bien entendu réalisée dans des conditions qui sont favorables à ladite hybridation, c'est-à-dire, et de manière évidente pour l'homme du métier, à un pH, à une température et dans une solution adéquats pour permettre l'hybridation des acides nucléiques. Cette solution constitue la solution à tester.

Selon l'invention, l'étape de lavage du support a notamment pour but d'éliminer les molécules en solution n'ayant pas réagi avec les molécules de reconnaissance sur le support, c'est-à-dire dans l'exemple ci-dessus les molécules d'acides nucléiques de la solution qui ne se sont pas hybridées aux molécules d'acides nucléiques fixées sur le support. Ceci permet d'éviter un fort bruit de fond qui serait généré par lesdites molécules non liées au support et non hybridées lors de la mesure.

Le lavage du support sur lequel sont fixées les molécules doit bien entendu être un lavage doux, qui ne dénature pas les acides nucléiques impliqués dans les hybridations, ne détruit pas lesdites hybridations formées, et ne détache pas les premières molécules fixées sur le support. Un exemple de lavage est donné dans les exemples ci-dessous. Cette étape de lavage permet d'obtenir l'échantillon de mesure utilisé pour mesurer l'absorption par la méthode photothermique choisie.

Selon le procédé de l'invention, lorsque la méthode photothermique choisie est une méthode de déflexion photothermique, on éclaire l'échantillon, comprenant par exemple des acides nucléiques, avec un faisceau lumineux appelé faisceau pompe et l'absorption du faisceau pompe par l'échantillon est détectée par la réfraction ou la réflexion d'un faisceau sonde.

Le faisceau pompe peut être par exemple un laser pulsé, ou un laser continu modulé en intensité. Il a de préférence une longueur d'onde d'émission dans la bande d'absorption de l'échantillon, par exemple dans celle des acides nucléiques lorsqu'il s'agit de détecter une hybridation d'acides nucléiques.

Selon l'invention, le faisceau sonde a de préférence une longueur d'onde qui n'est pas absorbée par le substrat ni par les molécules en présence.

Selon l'invention, le faisceau pompe peut être un faisceau d'un laser choisi parmi un laser argon continu à 275 nm, ou un laser YAG quadruplé de longueur d'onde 266 nm. Le faisceau pompe peut également être fourni par une source polychromatique, par exemple une lampe à vapeur de mercure, si le spectre d'émission de la source et son intensité permettent d'obtenir suffisamment de signal pour la détection.

Le faisceau sonde est dirigé à proximité de la portion d'échantillon éclairée par le faisceau pompe. Par ailleurs, le faisceau sonde peut être identique ou différent du faisceau pompe. Le faisceau sonde est de préférence à faisceau laser, par exemple celui d'un laser hélium à 633 nm.

La position relative des faisceaux sonde et pompe définit la configuration employée. Par exemple, le faisceau sonde peut traverser un ou plusieurs des milieux suivantes : l'échantillon, par exemple les oligonucléotides, le support solide, ou le milieu environnant, par exemple, un liquide ou de l'air. L'orientation du faisceau sonde par rapport au faisceau pompe peut être choisie à loisir, par exemple en fonction de l'encombrement mécanique et/ou pour optimiser la sensibilité en cherchant le maximum d'absorption en fonction de l'angle d'incidence.

Selon l'invention, les faisceaux sonde et pompe peuvent se croiser.

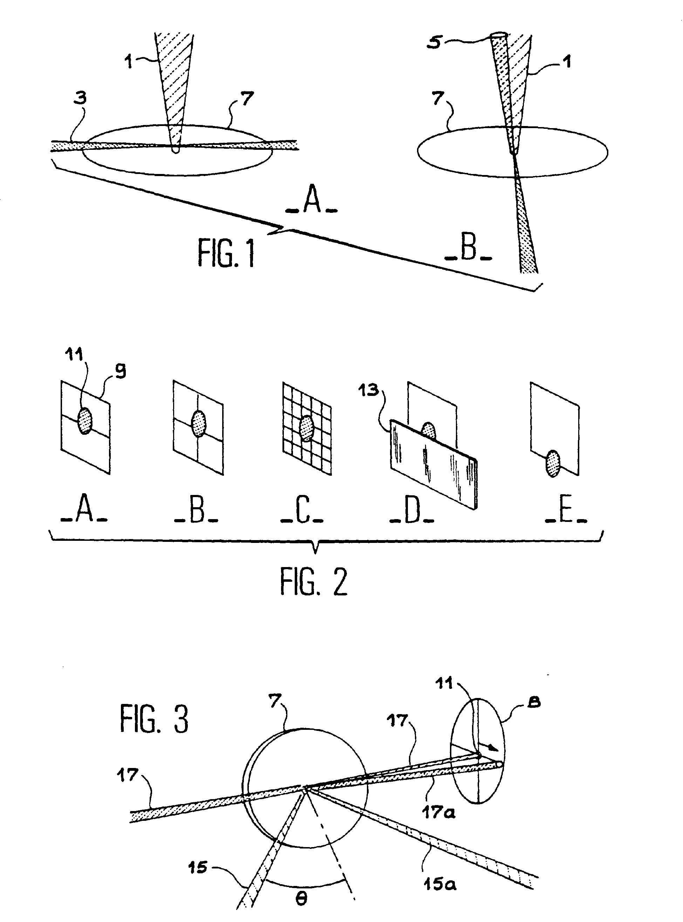

Selon l'invention, les faisceaux sonde et pompe peuvent être disposés dans une configuration transverse ou dans une configuration sensiblement colinéaire. Dans la configuration transverse, les faisceaux sonde et pompe se croisent et sont perpendiculaires. Cette configuration est représentée schématiquement sur la figure la annexée. Dans la configuration sensiblement colinéaire, les faisceaux pompe et sonde se croisent mais sont presque colinéaires. La figure 1b annexée est une représentation schématique de la configuration colinéaire.

Sur ces figures, la référence 1 indique le faisceau pompe, la référence 3 le faisceau sonde dans la configuration transverse, la référence 5 le faisceau sonde dans la configuration sensiblement colinéaire et la référence 7 l'échantillon de mesure, positionné sur le support solide.

La réflexion ou la réfraction du faisceau sonde peut être détectée au moyen d'une photodiode multi-élément, par exemple d'un détecteur à deux ou quatre quadrants, d'une barrette ou d'une matrice, ou à l'aide d'une photodiode simple, soit partiellement recouverte par un cache ou couteau, soit ne recevant qu'une partie du faisceau sonde.

Dans le cas d'une photodiode simple, un autre détecteur peut être nécessaire, afin de dissocier les variations d'absorption de l'échantillon des variations éventuelles de puissance du faisceau pompe.

La figure 2 est une illustration schématique de différentes configurations de détection de la déviation du faisceau sonde. Sur cette figure, -A- représente schématiquement un détecteur 9 bi-quadrant et un spot 11 formé par le faisceau sonde sur le détecteur, -B-représente un détecteur quatre quadrants, -C-représente un détecteur matriciel, -D- représente une photodiode simple partiellement recouverte d'un cache 13, et -E- représente un photodétecteur désaxé.

Pour obtenir une sensibilité suffisante, la déflexion du faisceau sonde induite par l'absorption d'une partie du faisceau pompe par l'échantillon, par exemple par les oligonucléotides, est de préférence distinguée des variations parasites dues à l'environnement, telles que les variations de température du laboratoire. Pour cela, le faisceau pompe peut être marqué temporellement, soit par une modulation s'il est continu, soit par son fonctionnement impulsionnel. La maîtrise de la fréquence de modulation ou de la possibilité d'obtenir un signal de référence permet de détecter préférentiellement la déflexion due à l'échauffement engendré par l'absorption partielle du faisceau pompe.

Quelle que soit la configuration des faisceaux utilisés, l'information obtenue est locale et concerne une zone autour du point d'impact du faisceau pompe sur l'échantillon, comprenant par exemple des acides nucléiques.

La taille de cette zone peut être fixée par les paramètres expérimentaux tels que la dimension du faisceau pompe au niveau du point d'impact sur l'échantillon ou sa fréquence de modulation, et par le comportement thermique du support, de l'échantillon et du milieu ambiant. Ceci est dû notamment à la diffusion de la chaleur dans ces différents milieux.

L'information étant locale, l'appareil de détection peut être couplé à un système de déplacement du support solide relativement au faisceau pompe. L'ensemble permet alors de comparer les valeurs de déviation du faisceau sonde d'un point à l'autre de l'échantillon, en particulier le signal peut être représenté sous forme de cartographie.

Par un point de l'échantillon, on peut par exemple comprendre une zone de reconnaissance moléculaire, c'est-à-dire une portion du support qui présente à sa surface des molécules présentant une propriété de reconnaissance d'un type donné de molécules cibles. Le support solide est donc associé à un échantillon, lui-même constitué de plusieurs points ou zones de reconnaissance. Les supports sont alors des supports fonctionnalisés.

Dans le cas des acides nucléiques, chacune de ces zones de reconnaissance est associée à une seule sorte de molécule de capture qui est préférentiellement, mais pas de manière limitative, différente des autres zones de reconnaissance qui constituent le même échantillon. Ainsi, il est possible soit d'augmenter la taille de chaque zone de reconnaissance, soit d'avoir plusieurs zones de reconnaissance associées à la même sorte de molécule de capture, et ce dans le but d'augmenter le signal à détecter.

Lorsque le signal est représenté sous la forme de cartographie, on a alors la mesure de plusieurs zones de reconnaissance, qui correspondent à plusieurs molécules cibles hybridées ou non sur les molécules de capture.

De plus, une ou plusieurs de ces zones de reconnaissance peuvent être affectés à un ou plusieurs échantillons de calibration évoqués précédemment.

Lorsque cela est nécessaire, le signal de déviation peut être converti en valeur d'absorption par exemple par l'intermédiaire d'un échantillon de référence ou échantillon de calibration. Cet échantillon de référence, dont l'absorption est connue et stable, peut faire l'objet d'une mesure de déflexion photothermique dans les mêmes conditions expérimentales que pour l'échantillon. La valeur obtenue permet par exemple de calculer un coefficient de conversion du signal électrique mesuré en niveau d'absorption.

Enfin, lorsque la méthode photothermique est une méthode de lentille thermique, on utilise un faisceau incident qui peut être un faisceau laser choisi parmi un laser argon continu à 275 nm, ou un laser YAG quadruplé de longueur d'onde 266 nm.

La méthode de lentille thermique est intéressante car le montage est plus simple.

En effet, dans ce cas, il n'y a qu'un seul laser qui fait office de laser sonde et de laser pompe. Après interaction avec l'échantillon, le point de focalisation est modifié. La mesure consiste simplement à mesurer le changement du flux lumineux au point initial. Le faisceau pompe peut être utilisé soit en transmission, c'est-à-dire à travers l'échantillon, soit en réflexion.

L'originalité de l'invention repose donc notamment sur le fait que jamais une technique photothermique n'a été utilisée pour la détection d'une reconnaissance moléculaire par exemple d'une hybridation d'oligonucléotides sur un support solide. Plus généralement, aucune méthode basée sur la mesure de variation d'absorption n'a été utilisée pour ce type de détection sur support.

Le procédé de la présente invention présente notamment l'avantage de ne nécessiter aucune étape de marquage ni marqueur. Il peut être utilisé par exemple avantageusement pour un test diagnostique par une détection d'hybridation d'acides nucléiques. Cette utilisation sera illustrée dans les exemples d'application ci-dessous.

De plus, il permet de détecter des absorptions et des variations d'absorption très faible, par exemple de quelques dixièmes de parties par million.

La présente demande décrit également un dispositif pour la mise en oeuvre du procédé de l'invention, ledit dispositif comprenant les éléments suivants :
- un moyen de positionnement du support solide,
- un moyen d'éclairage dudit support,
- un moyen de détection de l'absorption de la lumière par l'échantillon porté par le support, lorsqu'il est éclairé par ledit moyen d'éclairage, et
- un moyen de positionnement dudit moyen d'éclairage et dudit moyen de détection.

Selon l'invention, le moyen de positionnement du support peut être tout moyen connu de déplacement précis dudit support par exemple -des platines de translation et de rotation micrométrique par exemple de la marque de commerce MicroContrôle. Ces moyens peuvent être motorisés afin de permettre une automatisation notamment pour une cartographie.

Selon l'invention, les moyens d'éclairage de l'échantillon et de détection de l'absorption de la lumière par l'échantillon peuvent être choisis notamment en fonction de la méthode photothermique utilisée, du support et de la reconnaissance moléculaire à détecter. Le moyen d'éclairage de l'échantillon peut être par exemple un faisceau pompe tel qu'il est défini précédemment.

Lorsqu'il s'agit d'une méthode de déflexion photothermique, le moyen de détection de l'absorption peut comprendre un faisceau sonde et des moyens de détection de la réfraction ou de la réflexion d'un faisceau sonde. Ces moyens sont décrits ci-dessous et dans les exemples suivants.

Lorsqu'il s'agit d'une méthode de lentille thermique, le moyen de détection peut comprendre un faisceau sonde et un diaphragme placé au point de focalisation.

Selon l'invention, les moyens de positionnement des moyens d'éclairage et de détection précités peuvent être des moyens tels que ceux précités pour le positionnement du support.

D'autres éléments de l'invention et avantages apparaîtront encore à la lecture de la description et des exemples qui suivent en référence aux dessins en annexe, donnés bien entendu à titre illustratif et non limitatif.

### Brève description de l'annexe et des figures

La liste des séquences illustrant l'exemple 4 est donnée dans l'annexe.
- la figure 1 est une illustration schématique des configurations transverse (figure 1a) et colinéaire (figure 1b) des faisceaux pompe et sonde dans une détection par déflexion photothermique selon le procédé de l'invention ;
- la figure 2 est une illustration schématique de différentes configurations de détection de la déviation du faisceau sonde ;
- la figure 3 est une illustration schématique d'une mesure de l'absorption d'un échantillon par la méthode de déflexion photothermique selon l'invention ;
- la figure 4 est un schéma illustrant un dispositif pour la mise en oeuvre du procédé de la présente invention ;
- la figure 5 est un graphique illustrant des résultats de mesure de l'absorption d'un échantillon constitué d'acides nucléiques selon le procédé de la présente invention ;
- la figure 6 est une cartographie d'une détection selon le procédé de la présente invention réalisée sur un échantillon comportant deux rangées de plots d'oligonucléotides ;
- les figures 7a et 7b sont des histogrammes qui représentent le nombre de pixels de la cartographie de la figure 6.

### Exemples

### Exemple 1 : méthode de mesure

Le système utilisé selon l'invention est basé sur la déflexion photothermique en configuration transverse.

La figure 3 en annexe est une illustration schématique de principe d'une mesure de l'absorption d'un échantillon par la méthode de déflexion photothermique selon l'invention.

Sur cette figure, le faisceau pompe 15 est issu d'un laser argon continu à 275 nm (COHERENT de type INOVA 40 (marque de commerce), il est focalisé sur l'échantillon 7 à l'aide d'un miroir sphérique (non représenté), le diamètre du spot (non représenté) est d'environ 70 microns à la surface de l'échantillon 7. La longueur d'onde du faisceau pompe est choisie de manière à permettre la détection de l'hybridation d'acides nucléiques. La puissance du faisceau pompe est de 300 mW en sortie du laser.

Le faisceau sonde 17 est celui d'un laser Hélium-Néon à 633 nm. La longueur d'onde de ce faisceau sonde est indifférente. Selon l'invention, une longueur d'onde éloignée de celle du faisceau pompe permet d'obtenir un meilleur rapport signal sur bruit.

La détection de la déflexion est effectuée à l'aide d'un détecteur quatre quadrants -B- (voir figure 2) suivi d'une électronique d'amplification et de soustraction (non représentée).La référence 17a indique le faisceau sonde dévié par déflexion photothermique. L'angle θ indique l'angle d'incidence du faisceau pompe par rapport à la normale 20 à l'échantillon (indiquée en trait mixte).

Un filtre interférentiel (non représenté) sélectionnant la longueur d'onde du faisceau sonde peut être placé devant le détecteur quatre quadrants afin d'éviter l'influence d'une lumière parasite provenant du faisceau pompe modulé.

Dans le dispositif pour la mise en oeuvre de la présente invention, le laser sonde, le détecteur quatre quadrants et l'électronique associée peut faire partie intégrante d'une cellule de mesure commerciale par exemple celle de la société ALIS. Le signal issu de cette cellule est envoyé vers une détection synchrone.

Le faisceau pompe peut être modulé grâce à un disque à fente mécanique, appelé aussi ci-après chopper mécanique, dont la fréquence est réglable. Le signal de commande du chopper sert de référence à la détection synchrone. La fréquence est de 157 Hz. Le signal mesuré est obtenu à la sortie de la détection synchrone (amplitude du signal de déviation à la fréquence de modulation du faisceau pompe).

Le positionnement de l'échantillon et des deux faisceaux les uns par rapport aux autres est assuré par des platines de translation et de rotation (marque de commerce Micro-Contrôle, dont certaines sont motorisées afin de permettre une automatisation pour une cartographie, par exemple d'une biopuce, et dans certaines phases du réglage. Les réglages sont effectués automatiquement afin de maximiser le signal de déflexion dans un plan orthogonal à l'échantillon contenant le faisceau sonde. Lors des cartographies, si cela est nécessaire, un déplacement correctif est effectué dans une direction orthogonale aux axes de balayage de la cartographie afin de garantir la conservation d'un positionnement relatif correct au cours de la cartographie. Ce déplacement correctif est déterminé automatiquement dans une étape préliminaire de mesure. L'angle d'incidence du faisceau pompe par rapport à la normale à l'échantillon, et l'orientation de la cellule par rapport à l'échantillon, peuvent être réglables. Les positions et orientations relatives des faisceaux sonde et pompe peuvent être également réglables indépendamment.

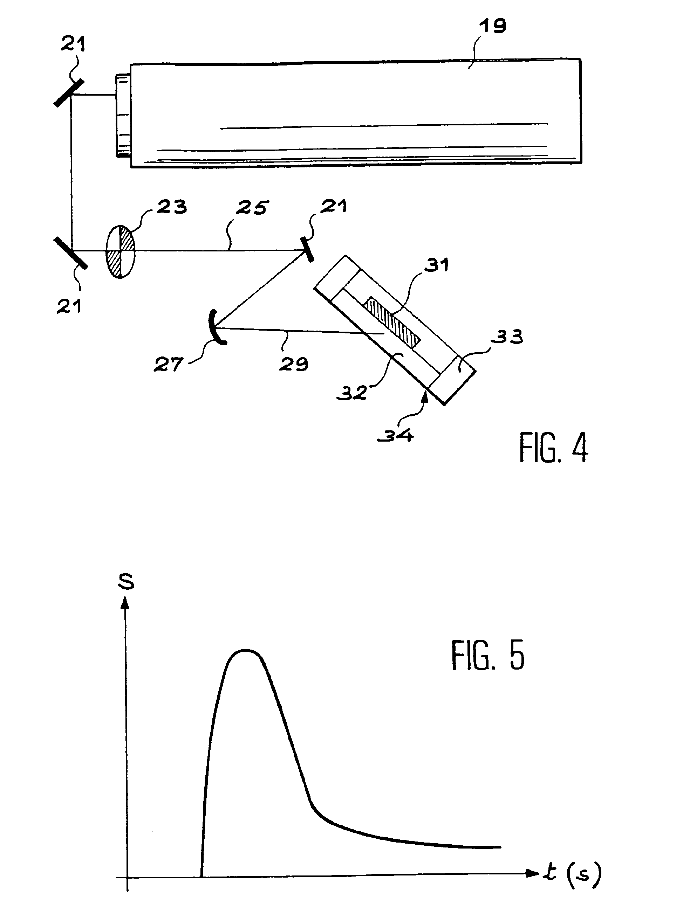

Un schéma d'un dispositif pour la mise en oeuvre de l'invention est représenté sur la figure 4 annexée. Sur cette figure, un obturateur, non représenté sur le schéma, permet de couper le faisceau pompe pendant les phases de déplacement et de le rétablir pendant un laps de temps bien déterminé après un temps d'attente choisi pour permettre la stabilisation du montage après un déplacement. La référence 19 indique un laser argon à 275 nm, la référence 21 des miroirs de positionnement du faisceau laser, la référence 23 indique un chopper mécanique, la référence 25 le faisceau laser après son passage à travers le chopper, la référence 27 un miroir de focalisation et la référence 31 l'échantillon de mesure.

La référence 34 constitue la cellule de mesure dans laquelle sont intégrés le laser sonde 32 et la diode à quatre quadrants 33.

La répétabilité du positionnement de l'échantillon peut être assurée par exemple par une lunette autocollimatrice qui n'est pas représentée sur le schéma. L'ensemble du dispositif de mesure peut être piloté par une station de travail, qui commande les déplacements et fait l'acquisition des signaux de déviation dans deux directions orthogonales.

Dans ce mode de réalisation, la déviation est mesurée suivant une direction parallèle au plan de l'échantillon et suivant une direction orthogonale à celui-ci. C'est cette dernière qui constitue le signal utile. Le signal électronique fourni par la détection synchrone peut être utilisé tel quel par comparaison d'un point à un autre de l'échantillon.

On peut également le convertir en valeur d'absorption en effectuant une mesure de référence sur un échantillon réputé stable dans le temps et sous flux laser, dont l'absorption est mesurable au spectrophotomètre et se situe dans la gamme de linéarité du banc de mesure de déflexion photothermique.

### Exemple 2 : Préparation des supports fonctionnalisés :

Les molécules choisies dans cet exemple sont des acides nucléiques, en particulier des oligonucléotides.

La réalisation des couches minces biologiques pour former l'échantillon de mesure selon le procédé de la présente invention peut par exemple comporter deux étapes :
1) fonctionnalisation du substrat, et
2) greffage des oligonucléotides.

### Fonctionnalisation du substrat

Le support choisi est en silice. Les oligonucléotides ne pouvant pas établir de liaison covalente avec ce type de matériau, ce dernier est fonctionnalisé. La fonctionnalisation du support a pour objet de modifier la surface du substrat afin d'y introduire des groupements réactifs qui permettront le greffage des oligonucléotides.

Cette étape requiert, par exemple, un premier traitement du support par exemple par un mélange sulfochromique puis une silanisation. Le traitement par le mélange sulfochromique peut être réalisé au moyen d'une solution saturée d'oxyde de chrome dans de l'acide sulfurique à 95%. Il a pour objectif d'éliminer les contaminants organiques présents sur le support et de générer des groupements silanols. La silanisation est ensuite réalisée par les techniques classiques de silanisation, par exemple par incubation du support dans une solution de toluène, aminopropyldiméthylethoxysilane (AMPMES) 1%. Durant cette étape, les silanes réagissent avec les groupements silanols générés au cours du traitement par le mélange sulfochromique. Il en résulte la formation d'une couche de silane porteuse de groupements amine sur lesquels il est possible de greffer des oligonucléotides.

### Greffage des oligonucléotides

Les oligonucléotides, qui constituent les molécules de capture, sont greffés sur le support soit directement, soit par l'intermédiaire d'une molécule d'avidine.

Dans ce dernier cas, une molécule d'avidine est préalablement greffée sur le silane par l'intermédiaire d'un agent de couplage, le phénylène-diisothiocyanate (PDC). L'avidine présente une forte affinité pour une petite molécule : la biotine. Elle peut donc reconnaître et fixer très fortement un oligonucléotide porteur d'une biotine. Le support est donc incubé dans une solution de diméthylformamide/pyridine 10% PDC 0,2%. Après lavage et séchage du support, l'avidine est déposée sous forme de gouttes de quelques millimètres de diamètre sur le support. Au même endroit est déposé l'oligonucléotide porteur d'une biotine.

### Exemple 3 : Utilisation des supports fonctionnalisés

Cette étape consiste à effectuer l'hybridation d'acides nucléiques ou de fragments de ceux-ci, qui constituent les molécules cibles présente dans la solution à tester. Elle consiste essentiellement en deux étapes :
1) hybridation des acides nucléiques (dans l'expérience décrite, un oligonucléotide complémentaire), et
2) lavage des acides nucléiques non hybridés.

Bien entendu préalablement à ces étapes et dans le cas particulier des acides nucléiques, il peut y avoir nécessité à effectuer d'autres étapes préparatoires pour obtenir la solution à tester. Ces étapes peuvent être des étapes d'extraction, d'amplification et de clivage.

### Extraction

Cette extraction est réalisée de manière tout à fait classique. En fait, on peut utiliser n'importe quelle technique d'extraction d'ADN, permettant d'obtenir du matériel susceptible d'être ultérieurement amplifié par un procédé d'amplification. Ces techniques de lyse des cellules, avec extraction puis purification des acides nucléiques sont habituellement celles recommandées pour des analyses génétiques, ou techniques rapides utilisant des produits commerciaux, tels que QIAmp Blood Kit (Marque déposée) de QIAGEN S.A.

### Amplification

Ainsi, il peut être judicieux d'augmenter le nombre de molécules cibles en amplifiant le signal. De très nombreuses techniques d'amplification existent. L'état de la technique décrit des méthodes permettant d'amplifier des séquences nucléotidiques utilisant des amorces spécifiques de ces séquences à amplifier. Ainsi on peut amplifier un fragment d'acide nucléique d'intérêt au sein d'une préparation d'acides nucléiques. De nombreuses techniques utilisent des oligonucléotides complémentaires de la séquence cible servant d'amorces pour l'élongation par une polymérase.

Pour l'amplification des ADN, il existe la PCR (Polymerase Chain Reaction), telle que décrite dans les brevets US-A-4,683,195, US-A-4,683,202 et US-A-4,800,159, la LCR (Ligase Chain Reaction), exposée par exemple dans la demande de brevet EP-A-0.201.184, ou la RCR (Repair Chain Reaction), décrite dans la demande de brevet WO-A-90/01069.

Pour l'amplification des ARN, plusieurs techniques ont aussi été décrites dans différents documents. Ces techniques sont les suivantes :
- 3SR (Self Sustained Sequence Replication) avec la demande de brevet WO-A-90/06995,
- NASBA (Nucleic Acid Sequence-Based Amplification) avec la demande de brevet WO-A-91/02818,
- SPSR (Single Primer Sequence Replication) avec le brevet US-A-5,194,370, et
- TMA (Transcription Mediated Amplification) avec le brevet US-A-5,399,491.

### Clivage

Le clivage peut également être judicieux car le produit des amplifications peut être constitué d'acides nucléiques de grandes tailles. Ainsi, lorsque l'on hybride de tels acides nucléiques cibles sur des molécules de capture, les duplex formés après hybridation avec les acides nucléiques cibles sont peu stables. C'est également le cas lorsque les polynucléotides sont utilisés en tant que sondes de détection. Les raisons peuvent être dues à la gêne stérique ou au manque de spécificité entre la molécule de capture, qui a été synthétisé, et sa molécule cible qui n'est pas forcément de même taille. Il va donc y avoir une perte quantitative et qualitative du signal.

La gêne stérique peut être le fait, non seulement, de la longueur de l'acide nucléique, mais également de l'existence ou de la conservation de structures secondaires. Le clivage permet de détruire ces structures et ainsi d'optimiser l'hybridation. Cette gêne stérique joue un rôle particulièrement important dans le cas de l'hybridation sur des surfaces contenant des sondes de capture à forte densité, par exemple les puces à ADN mises au point par la société Affymetrix ("Accessing Genetic Information with High-Density DNA arrays", M. Shee et al., Science, 274, 610-614. "Light-generated oligonucleotide arrays for rapide DNA sequence analysis", A. Caviani Pease et al., Proc. Natl. Acad. Sci. USA, 1994, 91, 5022-5026). Dans cette technologie, les sondes de capture sont généralement de tailles réduites, autour de vingt nucléotides.

En ce qui concerne le clivage des acides nucléiques, de nombreuses méthodes sont décrites dans l'état de la technique. Premièrement, le clivage peut être enzymatique, c'est-à-dire que la fragmentation des acides nucléiques peut être réalisée par des nucléases (DNases ou RNases). On génère alors des fragments de petites tailles avec des extrémités 3'-OH, 5'-OH, 3'-phosphate, 5'-phosphate.
Deuxièmement, le clivage peut être chimique. Par exemple dans le cas des ADN, on peut effectuer la dépurination ou la dépyrimidination des ADN, qui sont alors clivés en présence d'une base par un mécanisme dit de « β-élimination ». Le clivage des ADN peut être réalisé par des mécanismes d'oxydation, d'alkylation, d'addition de radicaux libres entre autres. Pour cliver les ARN, on utilise des cations métalliques souvent associés à des molécules organiques utilisées comme catalyseurs chimiques, par exemple l'imidazole. Ce clivage est préférentiellement réalisé en milieu alcalin et génère des fragments avec des extrémités 3'-phosphate.

### Hybridation

Le support sur lequel sont greffés les oligonucléotides est alors incubé dans une solution contenant l'oligonucléotide complémentaire par exemple une solution SSPE 6X + Triton X-100 à 0,05% + cible à 20 nM ; 30 min. à 35°C.

### Lavage des acides nucléiques non hybridés

Après hybridation, les acides nucléiques n'ayant pas hybridés sont éliminés par lavages dans la même solution SSPE 6X + Triton X-100 0,05%.

Juste avant la détection selon le procédé de la présente invention, les échantillons sont sortis de leur solution de lavage, passés rapidement sous l'eau pour éliminer le tampon en prenant garde de ne pas "déshybrider" les acides nucléiques, et séchés à l'air.

### Résultats

Une cartographie est réalisée sur un échantillon comportant deux rangées de plots d'oligonucléotides. Chaque plot fait environ trois millimètres de diamètre. Les plots de la rangée inférieure comportent des oligonucléotides 32mères hybridés par leur complémentaire alors que ceux de la rangée supérieure comportent un autre oligonucléotide 32mères, non complémentaire. Le pas de cartographie est de 250 microns.

Le temps de mesure est de 5 secondes par point dont une seconde avant ouverture de l'obturateur. Le point de mesure est donc illuminé pendant 4 secondes. Les mesures sont effectuées aussi rapidement que possible pendant cette durée. Le délai visé entre chaque mesure est d'un dixième de seconde. En chaque point de mesure, on dispose donc d'un signal dépendant du temps. L'allure de la variation du signal en fonction du temps est représentée par la courbe sur la figure 5 annexée.

Sur cette figure, sur l'ordonnée, S représente le signal en unité d'absorbance, et sur l'abscisse, t représente le temps en secondes.

Le signal comporte une période de bruit de fond, avant ouverture de l'obturateur, suivie d'une montée rapide après l'ouverture. Lorsqu'un maximum est atteint, le signal mesuré redescend, traduisant en cela l'évolution de l'échantillon au point de mesure. Pour comparer les signaux obtenus en chaque point de la cartographie, il a été préférable d'extraire de chaque signal une valeur qui puisse être affichée sous forme de cartographie. Cette valeur peut être par exemple le maximum mesuré ou la valeur à un temps de mesure donné. Il est également possible d'ajuster une fonction bien choisie à chaque signal temporel et choisir comme valeur représentative l'un des paramètres d'ajustement de la fonction ou le maximum de celle-ci. Après cette phase de traitement, le résultat est affiché sous forme de cartographie.

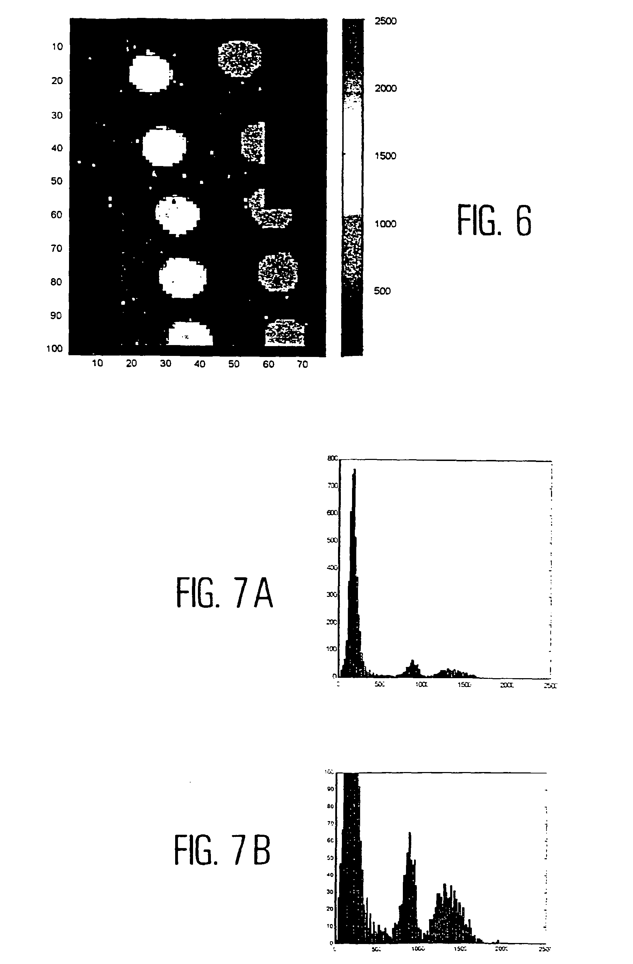

La cartographie que nous avons obtenue expérimentalement est donnée sur la figure 6 en annexe. Il s'agit des maxima du signal mesuré en chaque point de la cartographie, maxima ayant lieu pour t proche de 1,5 seconde. Sur cette cartographie, les plots d'oligonucléotides, hybridés ou non, se distinguent parfaitement du fond dont l'absorption est beaucoup plus faible. Les plots de la colonne de droite, de couleur bleu clair sur la cartographie, ont une absorption plus faible que ceux de la colonne de gauche, de couleur verte à jaune sur la cartograhie ; les rectangles noirs correspondent à des zones non cartographiées, car il y a eu un décalage manuel du support pendant l'acquisition de la cartographie pour mieux voir les plots de la colonne de droite. L'observation de la cartographie suffit donc pour déterminer si un plot d'oligonucléotide est hybridé ou non.

Les histogrammes des figures 7a et 7b en annexe représentent le nombre de pixels de la cartographie (axe des ordonnées) pour une intensité de signal donnée (axe des abscisses), à deux échelles différentes pour les ordonnées. On voit clairement apparaître trois modes, correspondant à l'absorption du fond, des oligonucléotides non hybridés, et des oligonucléotides hybridés. Ces modes sont bien séparés ce qui permet d'utiliser des méthodes de traitement d'image permettant de reconnaître automatiquement les plots hybridés ou non. Un simple seuillage permet par exemple de ne conserver que les plots hybridés et quelques points absorbants aisément distingués des plots de mesure.

### Exemple 4 : Test diagnostique

Une partie importante de la composante génétique de la susceptibilité à la polyarthrite rhumatoïde a pu être associée aux gènes HLA-DRB, codant pour la chaîne b des molécules HLA-DR impliquées dans la présentation des peptides aux lymphocytes T, fonction pivot au coeur des mécanismes de régulation de la réponse immunitaire. Il a été montré plus précisément que la présence d'une séquence particulière de cinq acides aminés, correspondant aux positions 70 à 74 de la troisième région hypervariable des molécules HLA-DRb1, était retrouvée pour différents allèles rapportés comme étant associés à la polyarthrite rhumatoïde. L'implication de cet « épitope partagé » correspondant aux séquences QKRAA ou QRRAA ou RRRAA (code à une lettre des acides aminés) est désormais bien documentée.

Toutefois, si ce groupe d'allèles DRB1*04 est effectivement associé à la polyarthrite rhumatoïde, tous les allèles actuellement connus (DRB1*0401 à DRB1*0427) ne sont pas forcément associés à cette maladie. Il peut en résulter que si l'on se limite à un typage du groupe d'allèles DRB1*04, en fonction des allèles présents, on pourra obtenir, outre de vrais positifs ou de vrais négatifs, des faux positifs qui vont alarmer inutilement le médecin et le patient.

Dans le cas d'une technique de biologie moléculaire (analyse des allèles DRB1 : résultats rendus selon la nomenclature DRB1*01 à DRB1*10), le clinicien s'intéresse essentiellement à la présence d'allèle(s) DRB1*04.

En cas de résultats suggérant une prédisposition à la maladie, un deuxième test est généralement pratiqué, utilisant une technique de biologie moléculaire dite de haute résolution dite de sous-typage du DR4, pour préciser l'allèle DRB1*04 (DRB1*0401 à 0427 selon la nomenclature officielle en 1998), seuls les allèles DRB1*0401, 0404, 0405 et 0408 étant rapportés comme étant associés à la maladie.

### Extraction

L'extraction est effectuée par des produits commerciaux évoqués précédemment, tels que QIAmp Blood Kit (Marque déposée) de QIAGEN S.A.

### Amplification

Cette amplification concerne le locus HLA-DR, incluant la région de l'exon 2 qui correspond aux codons 5 à 94 selon la nomenclature officielle, des gènes HLA-DRB. Le tableau 1 ci-dessous décrit les amorces utilisées lors de l'amplification par PCR de ce locus précédemment décrit. Il donne également l'ensemble des conditions physico-chimiques permettant la réalisation de cette amplification.

**Tableau 1 : amplification simultanée de la région d'intérêt HLA-DR**

| | |
|---|---|
| Amorces | - P1 (amorce 5'-DR) : CCG GAT CCT TCG TGT CCC CAC AGC ACG (5'>3') |
| | - P2 (amorce 3'-DR) : TCG CCG CTG CAC TGT GAA G (5'>3') |
| Mélange | - tampon 10X TEMAG (*) : 10 µl |
| réactionnel | - dNTPs (20 mM) : 1 µl (0.2 mM final) |
| | - P1 (30 µM) : 0.8 µl (0.25 µM final) |
| | - P2 (30 µM) : 0.8 µl (0.25 µM final) |
| | - AmpliTaq (5 U/µl) : 0.5 µl (2.5 U) |
| | - ADN : 100-500 ng |
| | - H₂O : QSP 100 µl |
| Programme d'amplification | (5 min à 96°C) + 10 x (10 sec à 98°C + 30 sec à 65°C + 30 sec à 72°C) + 30 x (20 sec à 96°C + 30 sec à 65°C + 30 sec à 72°C) |

| | |
|---|---|
| (*) : Tampon 10X TEMAG : 500 mM Tris-HCl pH 8.8 , 150 mM Sulfate Ammonium, 15 mM MgCl₂, 500 µM EDTA, 0.1 % gélatine | |

Dans cet exemple, la distance séparant les deux amorces P1 et P2 est choisie pour être suffisamment courte pour ne pas avoir à effectuer de clivage. Bien entendu, si les amplicons réalisés le nécessitent, cette fragmentation des acides nucléiques peut être effectuée selon les moyens et conditions exposés plus haut.

### Préparation des supports fonctionnalisés

Pour pouvoir réaliser une étude de prédisposition génétique à la polyarthrite rhumatoïde, il y a lieu d'utiliser un ensemble de réactions d'hybridation mettant en oeuvre un jeu de sondes oligonucléotidiques permettant l'analyse précise d'allèles ou de groupes d'allèles HLA-DRB1 et HLA-B*27. Le tableau 2 décrit l'ensemble des sondes qui est utilisé pour la détection de ces deux maladies. Les indications qui sont données de la gauche vers la droite sont les suivantes :
- la référence de la sonde attribué dans la nomenclature HLA,
- le numéro de la séquence attribué dans ce document,
- le gène HLA concerné,
- la séquence constituant cette sonde, et
- la localisation des codons (trois nucléotides) sur les gènes HLA.

**Tableau 2 : Sondes oligonucléotidiques**

| Sonde | SEQ ID NO | Gène HLA | Séquence (5'>3') | Localisation (codons) |
|---|---|---|---|---|
| C + | 1 | DR | TTC GAC AGC GAC GTG GGG | 40-45 |
| C - | 2 | - | TAT GAA ACT TAT GGG GAT AC | - |
| 4 | 3 | DR | GAT ACT TCT ATC ACC A | 29-34 |
| QK71 | 4 | DR | GAG CAG AAI CGG ICC GAG CAG AAG CGG GCC | 69-73 |
| IDE71 | 5 | DR | CTG GAA GAC GAI CGG CTG GAA GAC GAG CGG | 68-72 |
| E74 | 6 | DR | AGC AIA IGC IGG ICI AII AGC AGA GGC GGG CCG AGG | 69-75 |
| QR71 | 7 | DR | CAG AGG CGI GII ICI GTG CAG AGG CGG GCC GCG GTG | 70-75 |
| S57 | 8 | DR | GCC TAG CGC CGA GTA | 55-60 |
| 1 | 11 | DR | TGG CAG CTT AAG TTT GAA | 9-14 |
| 52 | 12 | DR | TAC TCT ACG TCT GAG T | 10-15 |
| 2 | 13 | DR | CAG CCT AAG AGG GAG TG | 10-15 |
| 7+9 | 14 | DR | IAG GTI GAC AIC GTG TGC CAG GTG GAC ACC GTG TGC | 74-79 |
| 10 | 15 | DR | GGA GGA GGT TAA GTT | 8-13 |
| 8+12 | 16 | DR | CTC TAC GGG IGA GT CTC TAC GGG TGA GT | 10-15 |
| 3 | 19 | DR | CCG GGT GGA CAA CIA C CCG GGT GGA CAA CTA C | 73-78 |

Pour certaines sondes, une deuxième séquence en caractères italiques précise la séquence naturelle, c'est-à-dire uniquement constituée des quatre nucléotides adénosine (A), thymine (T), guanine (G) et cytosine (C). Les autres séquences reprennent les mêmes nucléotides à l'exception de la substitution de certains par des nucléotides différents. Dans le cas présent, il s'agit de l'inosine. L'utilisation des inosines permet d'améliorer encore la spécificité des sondes vis-à-vis des séquences auxquelles elles vont s'hybrider. La spécificité des sondes de captures est bien précisée dans le tableau 3 ci-après.

**Tableau 3 : Spécificités principales des molécules ou sondes de capture**

| Sonde | SEQ ID NO | Spécificité |
|---|---|---|
| C + | 1 | Tous les DRB1* |
| C - | 2 | Aucun |
| 4 | 3 | DRB1 *04 |
| QK71 | 4 | DRB1 *0401, allèle possédant le motif QKRAA correspondant à l'épitope partagé |
| IDE71 | 5 | DRB1 *0402 |
| E74 | 6 | DRB1 *0403, 0406 et 0407 |
| QR71 | 7 | DRB1 *0101, 0404, 0405, 0408 et 1402, allèle possédant le motif QRRAA correspondant à l'épitope partagé |
| S57 | 8 | DRB1 *0405 |
| 1 | 9 | DRB1 *01 |
| 52 | 10 | DRB1 *03, 11, 13 et 14 |
| 2 | 11 | DRB1 *02 |
| 7+9 | 12 | DRB1 *07 et 09 |
| 10 | 13 | DRB1 *10 |
| 3 | 14 | DRB1 *03 |
| 8+12 | 15 | DRB1 *08 et 12 |

Ces sondes sont ensuite mises en place sur le support solide comme évoquées précédemment. Bien entendu pour faciliter l'analyse ultérieure, il convient de positionner chaque type de sonde sur un point ou une zone de reconnaissance.

Dans le cas présent. il y a nécessité d'avoir quinze zones de reconnaissance différentes, en incluant les contrôles, qu'ils soient positif ou négatif. Il est également possible d'effectuer d'autres tests sur un même support solide, il suffit pour cela d'augmenter le nombre de zones de reconnaissance.

**Tableau 4 : Organisation des différentes zones de reconnaissance sur le support solide**

| Zones de reconnaissance | | Zones de reconnaissance | |
|---|---|---|---|
| C + | SEQ ID NO 1 | 1 | SEQ ID NO 9 |
| C - | SEQ ID NO 2 | 52 | SEQ ID NO 10 |
| 4 | SEQ ID NO 3 | 2 | SEQ ID NO 11 |
| QK71 | SEQ ID NO 4 | 7 | SEQ ID NO 12 |
| IDE71 | SEQ ID NO 5 | 10 | SEQ ID NO 13 |
| E74 | SEQ ID NO 6 | 8+12 | SEQ ID NO 14 |
| QR71 | SEQ ID NO 7 | 3 | SEQ ID NO 15 |
| S57 | SEQ ID NO 8 | | |

Il y a un contrôle positif (SEQ ID NO 1), qui permet de détecter tous les allèles du gène DRB1*, ce qui permet de contrôler que l'amplification a bien concerné la région d'intérêt comprise entre les amorces P1 et P2 décrites dans le tableau 1. Le contrôle négatif (SEQ ID NO 2) n'a pas d'objectif diagnostic, il n'est présent que pour répondre à certaines normes. Cette séquence n'est absolument pas spécifique du HLA, et correspond à une séquence aléatoire non retrouvée chez les gènes HLA.

La SEQ ID NO 3 permet le typage de l'ensemble des allèles qui constitue le groupe DRB1*04. Elle permet l'identification de tous les allèles DRB1*04, allèles appartenant au groupe défini par des techniques de typage HLA par sérologie, comme le groupe DR4. Il s'agit d'une sonde à faible résolution, c'est-à-dire que de nombreux allèles peuvent être reconnus par celle-ci.

Les SEQ ID NO 4 à 8 permettent, quant à elles, le sous-typage de certains des allèles qui constituent le groupe DRB1*04, en précisant le ou les allèles partageant une séquence particulière. Il s'agit de sondes à haute résolution, c'est-à-dire que quelques allèles, voire un seul, peuvent être reconnus par une de ces sondes.

Toutes ces sondes sont utilisées pour détecter les allèles possédant l'épitope partagé associés à la prédisposition génétique à la polyarthrite rhumatoïde. Plus particulièrement, les sondes SEQ ID NO 4 et 7 permettent de détecter les allèles associés à la susceptibilité à la polyarthrite rhumatoïde, et les sondes SEQ ID NO 5 et 6 permettent de détecter les allèles associés à la résistance à ladite polyarthrite rhumatoïde. La sonde SEQ ID NO 8 permet de confirmer la présence d'un allèle DRB1*0405.

### Résultats

Dans les résultats ci-après exposés, on a travaillé avec des solutions à tester dont on connaissait les caractéristiques, afin de pouvoir contrôler si les résultats obtenus avec le procédé d'analyse, selon la présente invention, sont bien conformes à la réalité.

### 1^{ère} SOLUTION A TESTER :

Le tableau 5 qui suit, met en évidence les valeurs de signal obtenues avec la première solution à tester, auxquelles on a soustrait la valeur du signal de la sonde SEQ ID NO 2 (témoin négatif). Ces valeurs plus ou moins éloignées de la valeur zéro, permettent de déduire si l'hybridation a eu lieu ou non. La valeur 0 a été mise pour toutes les valeurs ne se distinguant pas significativement de la valeur obtenue pour SEQ ID NO 2 (écart inférieur à deux fois l'erreur standard à la moyenne calculée pour la sonde SEQ ID NO 2).

**Tableau 5 : Résultats d'hybridation obtenus avec la première solution à tester**

| Sonde associée à la zone de reconnaissance | Signal (unité arbitraire) | + / - | Sonde associée à la zone de reconnaissance | Signal (unité arbitraire) | +/- |
|---|---|---|---|---|---|
| SEQ ID NO 1 | 3000 | + | SEQ ID NO 9 | 0 | - |
| SEQ ID NO 2 | - | - | SEQ ID NO 10 | 0 | - |
| SEQ ID NO 3 | 920 | + | SEQ ID NO 11 | 2100 | + |
| SEQ ID NO 4 | 260 | + | SEQ ID NO 12 | 0 | - |
| SEQ ID NO 5 | 0 | - | SEQ ID NO 13 | 0 | - |
| SEQ ID NO 6 | 0 | - | SEQ ID NO 14 | 0 | - |
| SEQ ID NO 7 | 0 | - | SEQ ID NO 15 | 0 | - |
| SEQ ID NO 8 | 0 | - | | | |

L'analyse du HLA-DR montre que :
- la sonde SEQ ID NO 1 est positive : l'amplification HLA-DR et le test d'hybridation ont correctement fonctionné,
- les sondes SEQ ID NO 3 et SEQ ID NO 4 sont positives : un allèle DRB1*0401 est présent, et
- la sonde SEQ ID NO 11 est positive : un allèle DRB1*02 est présent.

En conclusion, il y a présence d'un allèle de susceptibilité à la polyarthrite rhumatoïde (DRB1* 0401), le second allèle étant DRB1* 02 qui fait preuve de neutralité vis-à-vis de la polyarthrite rhumatoïde.

Le typage HLA de ce premier échantillon était HLA-DRB1*0401 / 1602.

### 2^{ème} SOLUTION A TESTER :

Le tableau 6 qui suit, met en évidence les valeurs de DO obtenues avec la deuxième solution à tester.

**Tableau 6 : Résultats d'hybridation obtenus avec la deuxième solution à tester**

| Sonde associée à la zone de reconnaissance | Signal (unité arbitraire) | + / - | Sonde associée à la zone de reconnaissance | Signal (unité arbitraire) | +/- |
|---|---|---|---|---|---|
| SEQ ID NO 1 | 3000 | + | SEQ ID NO 9 | 0 | - |
| SEQ ID NO 2 | 0 | - | SEQ ID NO 10 | 0 | - |
| SEQ ID NO 3 | 310 | + | SEQ ID NO 11 | 2700 | + |
| SEQ ID NO 4 | 0 | - | SEQ ID NO 12 | 0 | - |
| SEQ ID NO 5 | 370 | + | SEQ ID NO 13 | 0 | - |
| SEQ ID NO 6 | 0 | - | SEQ ID NO 14 | 0 | - |
| SEQ ID NO 7 | 0 | - | SEQ ID NO 15 | 0 | - |
| SEQ ID NO 8 | 0 | - | | | |

Il y a quatre sondes positives, qui sont :
- la sonde SEQ ID NO 1 : l'amplification HLA-DR et le test d'hybridation ont bien fonctionné,
- la sonde SEQ ID NO 3 : il y a présence d'au moins un allèle DRB1* 04,
- la sonde SEQ ID NO 5 : il y a présence d'un allèle DRB1* 0402, et
- la sonde SEQ ID NO 11 : il y a présence d'un allèle DRB1*02.

Il s'agit donc de deux allèles DR4, non impliqués dans la susceptibilité génétique à la polyarthrite rhumatoïde DRB1*0402 et DRB1*02.

Le typage HLA de ce troisième échantillon était HLA-DRB1*0402 / 02.

### Références

**Doc.1 :**
   Balladur, V., Theretz, A., and Mandrand, B. Determination of the Main Forces Driving DNA Oligonucleotide Adsorption onto Aminated Silica Wafers. J.Colloid Interface Sci 194(2):408-418, 1997.
**Doc. 2 :**
   Cornell, B.A., Braach-Maksvytis, V.L.B., King, L.G., Osman, P.D.J., Raguse, B., Wieczorek, L., and Pace, R.J. A biosensor that uses ion-channel switches. Nature 387:580-583, 1997.
**Doc. 3 :**
   Elaïssari, A., Cros, P., Pichot, C., Laurent, V., and Mandrand, B. Adsorption of oligonucleotides onto negatively and positively charged latex particles. Colloids and Surfaces 83:25-31, 1994.
**Doc. 4 :**
   Guo, Z., Guilfoyle, A., Thiel, A.J., Wang, R., and Smith, L.M. Direct fluorescence analysis of genetic polymorphisms by hybridization with oligonucleotide arrays on glass supports. Nucl. Acids Res. 22(24):5456-5465, 1994.
**Doc. 5 :**
   Kohsaka, H., Taniguchi, A., Richman, D.D., and Carson, D.A. Microtiter format gene quantification by covalent capture of competitive PCR products: application to HIV-1 detection. Nucl.Acids Res. 21(15) : 3469-3472, 1993.
**Doc. 6 :**
   Maskos, U. and Southern, E.M. Oligonucleotide hybridisations on glass supports: a novel linker for oligonucleotide synthesis and hybridisation properties of oligonucleotides synthesised in situ. Nucl.Acids Res. 20(7):1679-1684, 1992.
**Doc. 7 :**
   Matson, R.S., Rampal, J.B., and Coassin, P.J. Biopolymer synthesis on polypropylene supports-I. Oligonucleotides. Analytical Biochemistry 217:306-310, 1994.
**Doc. 8 :**
   Noy, A., Vezenov, D.V., Kayyem, J.F., Meade, T.J., and Lieber, C.M. Stretching and breaking duplex DNA by chemical force microscopy. Chem.Biol. 4(7):519-527, 1997.
**Doc. 9 :**
   Pease, A.C., Solas, D., Sullivan, E.J., Cronin, M.T., Holmes, C.P., and Fodor, S.P. Light-generated oligonucleotide arrays for rapid DNA sequence analysis. Proceedings of the National Academy of Sciences USA 91:5022-5026, 1994.
**Doc. 10 :**
   Porath, J., Carlsson, J., Olsson, I., and Belfrage, G. Metal chelate affinity chromatography, a new approach to protein fractionation. Nature 258(5536):598-599, 1975.

### ANNEXE

### Liste de séquences

(1) INFORMATIONS GENERALES :
   (i) DEPOSANT :
      (A) NOM : BIOMERIEUX S.A.
      (B) RUE : Chemin de l'Orme
      (C) VILLE : Marcy-l'Etoile
      (E) PAYS : France
      (F) CODE POSTAL : 69280
      (G) TELEPHONE : (33) 78.87.20.00
      (H) TELECOPIE : (33) 78.87.20.90
   (ii) TITRE DE L'INVENTION : Procédé d'analyse de la prédisposition génétique d'un patient à au moins une maladie génétique
   (iii) NOMBRE DE SEQUENCES : 19
   (iv) FORME DECHIFFRABLE PAR ORDINATEUR :
      (A) TYPE DE SUPPORT : Floppy disk
      (B) ORDINATEUR : IBM PC compatible
      (C) SYSTEME D'EXPLOITATION : PC-DOS/MS-DOS
      (D) LOGICIEL : PatentIn Release #1.0, version #1.30 (OEB)
(2) INFORMATION SUR LA SEQ ID NO 1 :
   (i) CARACTERISTIQUES DE LA SEQUENCE :
      (A) LONGUEUR : 18 nucléotides
      (B) TYPE : acide nucléique
   (ii) TYPE DE MOLECULE : acide nucléique
   (iii) HYPOTHETIQUE : non
   (ix) CARACTERISTIQUES :
      (D) AUTRES INFORMATIONS : séquence issue du HLA DR
   (iv) DESCRIPTION DE LA SEQUENCE : SEQ ID NO 1 
TTCGACAGCG ACGTGGGG
10
(3) INFORMATION SUR LA SEQ ID NO 2 :
   (i) CARACTERISTIQUES DE LA SEQUENCE :
      (A) LONGUEUR : 20 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE : amorce
      (D) AUTRES INFORMATIONS :
   (xi) DESCRIPTION DE LA SEQUENCE : SEQ ID NO 2 
TATGAAACTT ATGGGGATAC
10 20
(4) INFORMATION SUR LA SEQ ID NO 3 :
   (i) CARACTERISTIQUES DE LA SEQUENCE :
      (A) LONGUEUR : 16 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 3 
GATACTTCTA TCACCA
10
(5) INFORMATION SUR LA SEQ ID NO 4 :
   (i) CARACTERISTIQUES DE LA SEQUENCE :
      (A) LONGUEUR : 15 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (B) AUTRES INFORMATIONS : séquence issue du HLA DR
   (xi) DESCRIPTION DE LA SEQUENCE : SEQ ID NO 4 
GAGCAGAAIC GG ICC
10
(6) INFORMATION SUR LA SEQ ID NO 5 :
   (i) CARACTERISTIQUES DE LA SEQUENCE :
      (A) LONGUEUR : 15 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 5 
CTGGAAGACG AICGG
10
(7) INFORMATION SUR LA SEQ ID NO 6 :
   (i) CARACTERISTIQUES DE LA SEQUENCE :
      (A) LONGUEUR : 18 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE : amorce
      (B) AUTRES INFORMATIONS : séquence issue du HLA DR
   (xi) DESCRIPTION DE LA SEQUENCE : SEQ ID NO 6 
AGCAIAIGCI GGICIAII
10
(8) INFORMATION SUR LA SEQ ID NO 7 :
   (i) CARACTERISTIQUES DE LA SEQUENCE :
      (A) LONGUEUR : 18 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 7 
CAGAGGCGIG IIICIGTG
10
(9) INFORMATION SUR LA SEQ ID NO 8 :
   (i) CARACTERISTIQUES DE LA SEQUENCE :
      (A) LONGUEUR : 15 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 8 
GCCTAGCGCC GAGTA
10
(12) INFORMATION SUR LA SEQ ID NO 9 :
   (i) CARACTERISTIQUES DE LA SEQUENCE :
      (A) LONGUEUR : 18 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 11 
TGGCAGCTTA AGTTTGAA
10
(13) INFORMATION SUR LA SEQ ID NO 10 :
   (i) CARACTERISTIQUES DE LA SEQUENCE :
      (A) LONGUEUR : 16 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 12 
TACTCTACGT CTGAGT
10
(14) INFORMATION SUR LA SEQ ID NO 11 :
   (i) CARACTERISTIQUES DE LA SEQUENCE :
      (A) LONGUEUR : 17 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 13 
CAGCCTAAGA GGGAGTG
10
(15) INFORMATION SUR LA SEQ ID NO 12 :
   (i) CARACTERISTIQUES DE LA SEQUENCE :
      (A) LONGUEUR : 18 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 14 
IAGGTIGACA ICGTGTGC
10
(16) INFORMATION SUR LA SEQ ID NO 13 :
   (i) CARACTERISTIQUES DE LA SEQUENCE :
      (A) LONGUEUR: 15 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 15 
GGAGGAGGTT AAGTT
10
(17) INFORMATION SUR LA SEQ ID NO 14 :
   (i) CARACTERISTIQUES DE LA SEQUENCE :
      (A) LONGUEUR : 14 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 16 
CTCTACGGGI GAGT
10
(18) INFORMATION SUR LA SEQ ID NO 15 :
   (i) CARACTERISTIQUES DE LA SEQUENCE :
      (A) LONGUEUR : 16 nucléotides
      (B) TYPE : acide nucléique
   (ii) TYPE DE MOLECULE : acide nucléique
   (iii) HYPOTHETIQUE : non
   (ix) CARACTERISTIQUES :
      (A) NOM/CLE :
      (D) AUTRES INFORMATIONS : séquence issue du HLA DR
   (xi) DESCRIPTION DE LA SEQUENCE : SEQ ID NO 19

CCGGGTGGAC AACIAC
10

## Revendications

1. Procédé de détection sans marquage d'une réaction de reconnaissance moléculaire entre une première molécule fixée sur un support, et une deuxième molécule présente dans une solution à tester, dans lequel la détection est réalisée par une méthode photothermique.

2. Procédé de détection sans marquage d'une réaction d'hybridation d'acides nucléiques entre une première et une deuxième molécules d'acides nucléiques comprenant les étapes suivantes :
- fixation de la première molécule d'acide nucléique sur un support solide,
- mise en contact de la première molécule d'acide nucléique fixée sur le support solide avec une solution à tester soupçonnée de comprendre la deuxième molécule d'acide nucléique, cette dernière étant apte à s'hybrider à ladite première molécule, la mise en contact étant réalisée dans des conditions favorables à ladite hybridation,
- lavage du support solide pour isoler un échantillon de détection constitué de ladite première molécule fixée sur le support et éventuellement de ladite deuxième molécule liée à la première molécule, et
- mesure de l'absorption de l'échantillon par une méthode photothermique.

3. Procédé selon la revendication 1 ou 2, dans lequel la méthode photothermique est une méthode de lentille thermique.

4. Procédé selon la revendication 1 ou 2, dans lequel la méthode photothermique est une méthode de déflexion photo-thermique dans laquelle l'échantillon est éclairé par un faisceau pompe, et l'absorption du faisceau pompe par l'échantillon est détectée par la réfraction ou la réflexion d'un faisceau sonde.

5. Procédé selon la revendication 4, dans lequel les faisceaux sonde et pompe se croisent.

6. Procédé selon la revendication 4, dans lequel les faisceaux sonde et pompe sont dans une configuration transverse ou dans une configuration sensiblement colinéaire.

7. Procédé selon l'une quelconque des revendications 4 à 6, dans lequel le faisceau pompe est choisi parmi un laser pulsé, un laser continu modulé en intensité, ou une lumière polychromatique.

8. Procédé selon l'une quelconque des revendications 4 à 7, dans lequel la réfraction ou la réflexion du faisceau sonde est détectée au moyen d'une photodiode multi-éléments ou à l'aide d'une photodiode simple ne recevant qu'une partie du faisceau sonde.

9. Procédé selon l'une quelconque des revendications 4 à 8, dans lequel le faisceau pompe est un faisceau d'un laser choisi parmi un laser argon continu à 275 nm, un laser YAG quadruplé de longueur d'onde 266 nm ou une lumière polychromatique.

10. Procédé selon l'une quelconque des revendications 4 à 9, dans lequel le faisceau sonde a une longueur d'onde qui n'est pas absorbée par le substrat ni les molécules en présence.

11. Procédé selon la revendication 3, dans lequel un faisceau incident est utilisé, ledit faisceau étant un faisceau d'un laser choisi parmi un laser argon continu à 275 nm, un laser YAG quadruplé de longueur d'onde 266 nm ou une lumière polychromatique.

12. Procédé selon l'une quelconque des revendications précédentes, comprenant en outre une étape de comparaison de la mesure de l'absorption de l'échantillon avec celle d'un échantillon témoin.

13. Utilisation de procédé selon l'une quelconque des revendications 1 à 12 pour un test, un diagnostic ou une détection d'hybridation d'acides nucléiques.

## Claims

1. Detection method of a molecular recognition reaction between a first molecule fixed on a support and a second molecule present in a solution to be tested, without labelling, in which the detection is made by a photothermal method.

2. Detection method of a hybridization reaction of nucleic acids between a first and a second molecules of nucleic acids, without labelling, consisting in the following steps:
- fixing the first nucleic acid molecule on a solid support,
- contacting the first nucleic acid molecule fixed on the solid support with a solution to be tested suspected of containing the second nucleic acid molecule, this latter being capable of being hybridized with said first molecule, the contacting being carried out under conditions favourable for said hybridization,
- washing the solid support to isolate a detection sample formed from said first molecule fixed on the support and possibly said second molecule hybridized on said first molecule, and
- measuring the absorption of the sample by a photothermal method.

3. Method according to Claim 1 or 2, in which the photothermal method is a thermal lens method.

4. Method according to Claim 1 or 2, in which the photothermal method is a method of photothermal deflection in which the sample is illuminated by a pump beam and the absorption of the pump beam by the sample is detected by the refraction or the reflection of a probe beam.

5. Method according to Claim 4, in which the probe and pump beams intersect.

6. Method according to Claim 4, in which the probe and pump beams are in a transverse configuration or in a substantially collinear configuration.

7. Method according to any one of Claims 4 to 6, in which the pump bean is chosen from a pulsed laser, a continuous intensity modulated laser or polychromatic light.

8. Method according to any one of Claims 4 to 7, in which the refraction or the reflection of the probe beam is detected by means of multielement photodiode or by means of a simple photodiode receiving only part of the probe beam.

9. Method according to any one of Claims 4 to 8, in which the pump beam is a beam from a laser chosen from a continuous argon laser at 275 nm, a quadrupled YAG laser with a wavelength of 266 nm or a polychromatic light.

10. Method according to anyone of Claims 4 to 9, in which the probe beam has a wavelength that is absorbed neither by the substrate nor by the present molecules.

11. Method according to Claim 3, in which an incident beam is used, said beam being a beam from a laser chosen from a continuous argon laser at 275 nm, a quadrupled YAG laser with a wavelength of 266 nm or a polychromatic light.

12. Method according to any one of the preceding claims, comprising in addition a step for comparing the absorption measurement of the sample with that of a control sample.

13. Use of a method according to any one of Claims 1 to 12 for a test, a diagnosis or a detection of hybridization of nucleic acids.

## Patentansprüche

1. Verfahren zum Nachweis ohne Markierung einer molekularen Erkennungsreaktion zwischen einem ersten, auf einem Träger fixierten Molekül und einem zweiten, in einer zu untersuchenden Lösung vorliegenden Molekül, wobei der Nachweis durch ein photothermisches Verfahren durchgeführt wird.

2. Verfahren zum Nachweis ohne Markierung einer Hybridisierungsreaktion von Nucleinsäuren zwischen einem ersten und einem zweiten Nucleinsäuremolekül, das die folgenden Schritte umfasst:
- Fixierung des ersten Nucleinsäuremoleküls auf einem festen Träger,
- In-Kontakt-Bringen des ersten, auf dem festen Träger fixierten Nucleinsäuremoleküls mit einer zu untersuchenden Lösung, von der vermutet wird, dass sie das zweite Nucleinsäuremolekül enthält, wobei sich letztere mit dem ersten Molekül zu hybridisieren vermag und das In-Kontakt-Bringen unter für die Hybridisierung förderlichen Bedingungen durchgeführt wird,
- Waschen des festen Trägers zum Isolieren einer Nachweisprobe, die sich aus dem ersten, auf dem Träger fixierten Molekül und gegebenenfalls dem zweiten, an das erste Molekül gebundenen Molekül zusammensetzt, und
- Messen der Absorption der Probe durch ein photothermisches Verfahren.

3. Verfahren gemäß Anspruch 1 oder 2, wobei das photothermische Verfahren ein Wärmelinsenverfahren ist.

4. Verfahren gemäß Anspruch 1 oder 2, wobei das photothermische Verfahren ein photothermisches Deflexionsverfahren ist, bei dem die Probe mit einem Anregungsstrahl bestrahlt wird und die Absorption des Anregungsstrahls durch die Probe durch die Brechung oder die Reflexion eines Sondenstrahl ermittelt wird.

5. Verfahren gemäß Anspruch 4, wobei sich Sonden- und Anregungsstrahl kreuzen.

6. Verfahren gemäß Anspruch 4, wobei Sonden- und Anregungsstrahl transversal angeordnet sind oder im Wesentlichen kollinear angeordnet sind.

7. Verfahren gemäß einem der Ansprüche 4 bis 6, wobei der Anregungsstrahl aus einem Impulslaser, einem intensitätsmodulierten Dauerlaser oder polychromatischem Licht ausgewählt ist.

8. Verfahren gemäß einem der Ansprüche 4 bis 7, wobei die Brechung oder die Reflexion des Sondenstrahls mittels einer Mehrelementphotodiode oder mit Hilfe einer einfachen Photodiode ermittelt werden, die nur einen Teil des Sondenstrahls empfängt.

9. Verfahren gemäß einem der Ansprüche 4 bis 8, wobei der Anregungsstrahl ein Strahl eines Lasers ist, der aus einem Argondauerlaser mit 275 nm, einem frequenzvervierfachten YAG-Laser mit einer Wellenlänge von 266 nm oder polychromatischem Licht ausgewählt ist.

10. Verfahren gemäß einem der Ansprüche 4 bis 9, wobei der Sondenstrahl eine Wellenlänge aufweist, die weder vom Substrat noch den vorhandenen Molekülen absorbiert wird.

11. Verfahren gemäß Anspruch 3, wobei ein einfallender Strahl verwendet wird und der einfallende Strahl ein Strahl eines Lasers ist, der aus einem Argondauerlaser mit 275 nm, einem frequenzvervierfachten YAG-Laser mit einer Wellenlänge von 266 nm oder polychromatischem Licht ausgewählt ist.

12. Verfahren gemäß einem der vorangehenden Ansprüche, das außerdem einen Schritt des Vergleichs der Höhe der Absorption der Probe mit der einer Vergleichsprobe umfasst.

13. Anwendung des Verfahrens gemäß einem der Ansprüche 1 bis 12 bei einem Test, einer Diagnose oder einem Nachweis einer Nucleinsäurenhybridisierung.
